# EUROPEAN PATENT SPECIFICATION

(11) **EP 2 505 228 B1**
(45) Date of publication and mention of the grant of the patent: **21.11.2018**
(21) Application number: 12162608.9
(22) Date of filing: 02.02.2005
(51) Int. Cl.: A61N 1/05, A61F 2/48

(54) **An improved percutaneous lead**
Verbesserte perkutane Elektrode
Electrode-sonde percutanée améliorée

(30) Priority: 04.02.2004 AU 2004900538
(43) Date of publication of application: 03.10.2012
(62) Divisional of application: 05700165.3
(73) Proprietor: TC1 LLC, St. Paul, MN 55117 (US)
(72) Inventor: Ayre, Peter Joseph, Crows Nest, New South Wales 2065 (AU); Woodard, John Campbell, Thornleigh, New South Wales 2120 (AU); Begg, John Donald, Nashdom Lane, Burnham Slough SL1 8NJ (GB)
(74) Representative: ZBM Patents - Zea, Barlocci & Markvardsen

(56) References cited:
- WO-A1-95/17026
- WO-A1-02/094348
- US-A- 3 699 956
- US-A- 5 849 033
- US-A- 5 904 646
- US-A1- 2002 058 978

## Description

### Field of Invention

The present invention relates to an improved percutaneous lead and improved means of implanting said lead for use with implantable medical devices.

### Background

A substantial amount of medical research is currently being aimed at treating disease by the use of implantable medical assist devices. Some of these implantable medical assist devices passively assist patient's body functions. Examples of passive medical devices include: artificial cannulation to replace or assist failing arteries or veins; and various artificial implants such as artificial blood implants. Other implantable medical devices are called active implantable medical devices. These active implantable medical devices generally require a power source or supply to function or aid the patient's normal bodily functions. These active implantable medical devices may include pacemakers, implantable pumps, neuro-stimulators, and cochlear implants.

There has been a long felt need to be able to safely and reliably implant active medical assist devices and to avoid long term patient problems associated with the use of such devices. One of the common problems encountered with the use of these devices is that a substantial proportion of these generally require a means of communicating electrical information, data, and/or power with the external environment outside the body of a patient, when implanted.

The traditional solution for this problem is to connect the implanted active medical device to a percutaneous lead. This lead preferably extends from the implanted device within the patient's body, through the skin layer of a patient then to a controller, computer or power circuit (external to the patient's body). This traditional configuration may lead to increased risk of bacterial infection and reduced quality of life for the patient. Additionally there is a risk that said lead may be accidentally severed by the patient and this raises safety and reliability concerns relating to the traditional use of percutaneous leads.

In the past, there have been other inventions aimed at reducing or eliminating the need for a permanent wound at the lead's exit site in the patient's skin layer. These other inventions used RF transceiver devices mounted internally and externally in relation to the patient to relay electrical signals without the need for a hole in the patient's skin. These RF transceiver devices may cause significant damage or physical harm to the patient due to: adverse heating events to the patient's internal organs, reductions in a patient's quality of life, burns, discomfort and also transmission efficiency problems with the quality of the data and power transceived by such systems. All of these problems lead to inevitable safety and reliability relating to use of such systems by patients.

The present invention aims at addressing or ameliorating at least some of the aforementioned problems of the prior art. US5904646 dislcoses a bone-mounted percutaneous lead to bring electric power to an implanted electrical device.

### Brief description of the invention

The present invention is set out in the appended claims. Described herein, in a broad form, is a percutaneous lead assembly for supplying electrical signals to a medical device implanted within a body of a patient, said lead assembly comprising a flexible elongate member having a first portion adapted to remain external to the body of a patient, said first portion having a first diameter; and a second portion joined to said first portion and adapted to extend through a hole in a skin layer of the body of the patient, and wherein said second portion having a second diameter which is substantially smaller than said first diameter.

Said first portion includes a shielding layer. Additionally, at least a segment of said second portion is covered with a textured surface.

Said first portion and said second portion are joined by connectors. In some examples, said percutaneous lead assembly may include a lead restraint.

Preferably said external lead restraint is implanted within a body of a patient and extends through a hole in the patient's skin and characterised in that an excess length of lead is releasably secured near to the hole by releasable securing means affixed to the patient's skin.

Described herein is a percutaneous lead assembly for supplying electrical signal to a medical device implanted within a body of a patient, wherein said lead assembly has a flexible elongate member including a first unshielded portion that extends through a hole in a skin layer of the body of the patient; and a second shielded portion which is joined to said first unshielded portion at a site external to the body of the patient.

### Brief description of the drawings

Embodiments of the present invention will now be described with reference to the accompanying drawings wherein:
Figure 1 shows a schematic view of a first preferred embodiment of the present invention, in situ;
Figure 2 shows a cut away side view of a portion of a preferred embodiment;
Figure 3 shows a cut away side view of a portion of a preferred embodiment;
Figure 4 shows a cross sectional side view of an embodiment;
Figure 5 shows a top view of a preferred embodiment; and
Figure 6 shows a cross sectional side view of a portion of the strain terminator mechanism shown in figure 5.

### Brief description of the preferred embodiments

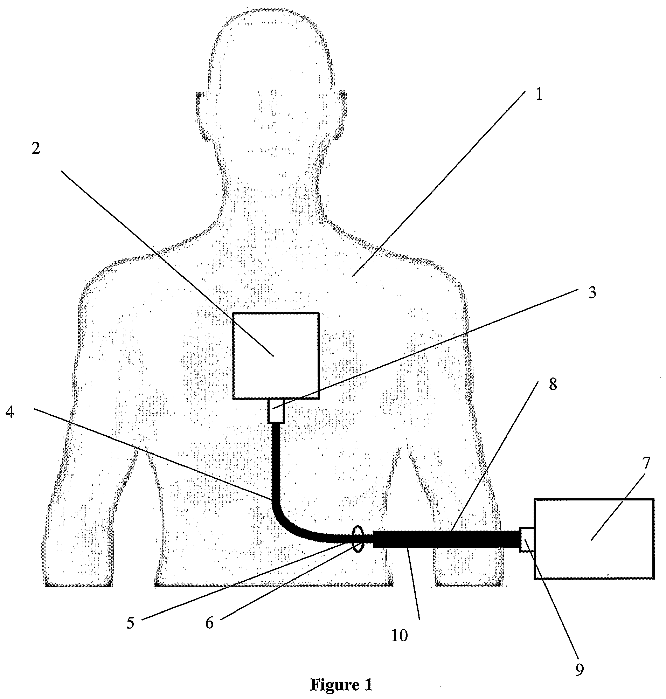

The present invention generally relates to an improvement to percutaneous lead assemblies. A first preferred embodiment of this invention is shown in Figure 1. In this embodiment, a patient 1 is implanted with a medical assist device 2 to assist or enhance the patient's body function. Preferably, this medical assist device may be active or passive and may require uni- or bi-directional data, instructions, and/or power in the form of electrical signals from the external environment. Preferably, these electrical signals may be communicated by an external controller 7. Please note that it may be preferable to use this embodiment in conjunction with an implantable blood pump or a left ventricle assist device.

In the embodiment shown in Figure 1, the external controller 7 is in electrical communication with the implanted medical device 2 by the use of the flexible percutaneous lead assembly 10. The external controller 7 is or may include any of the following devices: batteries, power supply, hardware controller, personal computer, microcontroller, and/or microprocessors.

The connection formed by the percutaneous lead assembly 10 may allow for the transmission and reception of electrical signals. The lead assembly 10 may allow for a continuous electrical link between the medical device 2 and the controlling device 7 by the use of continuous wiring (not shown in Figure 1) running through the core of the lead assembly 10. Preferably, the lead assembly 10 extends from the medical device 2, implanted within the body of the patient 1, through a hole or aperture 5, made by a physician or doctor, to the controlling device 7.

The preferred percutaneous lead assembly 10 may also include: two ends, two connectors 3 & 9, wherein one connecter is connected to either end of the lead assembly 10 and wherein preferably each connector 3 & 9 is designed to mate with a respective corresponding connector on the medical device 2 and/or the controlling device 7.

The percutaneous lead assembly 10 has a first portion 8 and a second portion 4. The second portion 4 may extend from the first connector 3 through the aperture 5 and join with the first portion 8. Preferably, the section of the lead referred to as the second portion 4 may include regions coated with a textured surface. This textured surface may be produced by coating the region of the lead with velour or Dacron™. These types of coating materials promote ingrowth of the patient's cells into the surface of the textured surface and assist in anchoring lead assembly 10 within the patient's body 1. It is also preferred to only coat the lead portions, where necessary to achieve the desired amount of ingrowth or anchoring within the body 1.

Additionally, the second portion 4 extends out from the patient's body 1 through the hole 5. This extension past the hole 5 is shown by relatively thin region 6. Preferably, region 6 does not include a textured coating. Please note that hole 5 may also be referred to as a permanent exit wound.

In this embodiment, the relatively thin region 6 is integrally joined to the relatively thick region of first portion 8. The first portion 8 is also joined to a connector 9. When in use, the connector 9 may be connected to a controlling device 7.

The second portion 4 passing through the exit wound 5 generally allows the exit wound to be of a substantially smaller diameter than otherwise would be the case if the lead assembly was of a uniform thickness. This reduction in the size of the exit wound may lessen the trauma experienced by patient 1 during and after implantation; as well as reducing the chance of infection at or near to the exit wound region. The relatively thick region of first portion 8 of the lead assembly 10 may allow for increased wear resistance of the external portion of the lead as well as providing extra shielding for the wiring within the assembly 10.

Please also note that the first portion 8 may be constructed by wrapping or coating the relatively thin regions that extend externally from the patient's body and effectively protect or reinforce the external portion of the lead assembly 10. Additionally, a protective sheath may be used with the first portion 8 to achieve a similar effect of protecting the external portion of the wiring assembly.

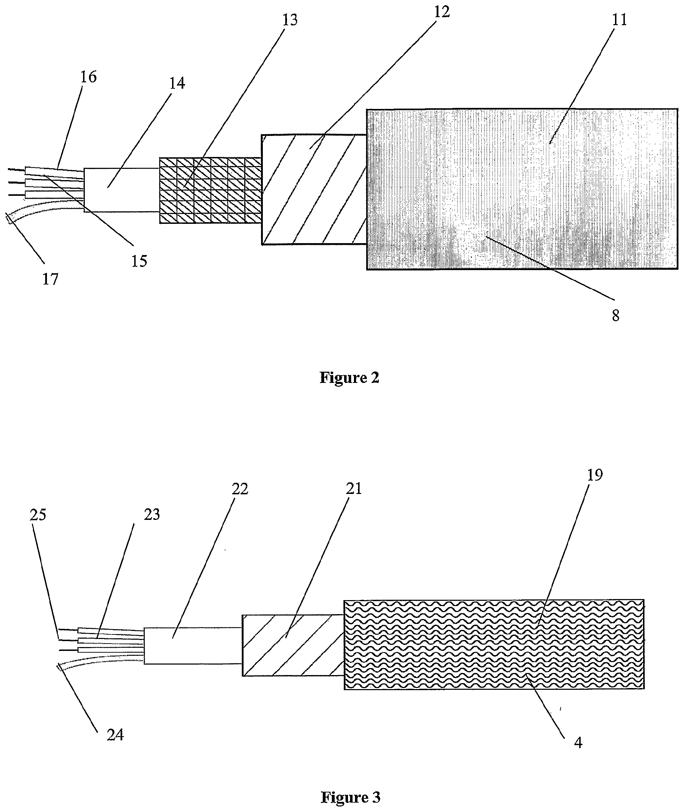

A preferred embodiment shown in Figure 2 depicts a cross sectional cut away view of the first portion 8 of lead assembly 10. In this embodiment, the first portion 8 of the lead assembly 10 may include: an outer protective sheath 11, an inner protective sheath 12, an electromagnetic shielding layer 13, and a wire bundle 14.

Preferably, the outer protective sheath 11 is constructed from a tough but flexible material that is preferably wear resistant and/or cut resistant. The outer protective sheath 11 may be constructed of polyurethane material. Please note that the materials used to construct the first portion 8 of the lead assembly do not need to be biocompatible and may even be toxic during implant conditions. This is because the first portion 8 is preferably not implanted within the body of the patient.

The inner protective sheath 12 provides additional wear resistance. Generally, the inner protective sheath 12 may function to support the general shape and configuration of the first portion 8. Preferably, the inner protective sheath 12 is flexible yet resistant to wear. In some preferred embodiments of the present invention, the inner protective sheath 12 may be constructed of silicone rubber or a similar polymer known as Nusil™. Silicone and Nusil™ also have the advantage that they are relatively transparent and enable easy inspection as to the condition and quality of the inner protective sheath 12.

The electromagnetic shielding layer 13 is included within the structure of the first portion 8 of the lead assembly. This layer may function to prevent electromagnetic interference from the outside environment interfering with the electric signals being communicated by the lead assembly, when in use. The electromagnetic shielding layer 13 is preferably constructed from braided stainless steel and this is because metals generally provide the most efficient electromagnetic shielding. Additionally, stainless steel braid is relatively wear resistant and cut resistant, which prevents accidental breakage by a patient, user or doctor. Also, stainless steel is generally resistant to oxidation or rusting and is therefore preferred for long term applications in vigorous environments and is also suitable for implantation.

Within the electromagnetic shielding layer 13 may be a wire bundle 14 which contains the wires to act as an electrical conduit for the lead assembly. The wire bundle is generally assembled by inter weaving several insulated wires 15 with each other and a wiring strain relief 17. The position of the wires and the mechanical strain relief set in place using second layer of silicone or Nusil™. Preferably, the lead assembly 10 includes three wires, but any number of wires are possible. An increase in the number of wires will increase the overall minimum diameter of the lead assembly, therefore it is preferred to include a minimum amount of insulated wires to provide functionality to the implantable medical device for which the lead assembly is to cooperate.

Preferably, the wiring strain relief 17 is constructed from 2 Kevlar™ cords with a combined approximate breaking strain of 630N. Additionally, the wires 16 within the wire bundle 14 should be separately insulated preferably using Perfluoroalyoxy ('PFA') insulation 15.

A further embodiment is shown in figure 3. This figure depicts the second portion 4 of the lead assembly 10. The second portion 4 may include a textured outer surface 19, outer protective layer 21, and a wire bundle 22.

Preferably, at least a segment of second portion 4 is covered with a textured outer surface 19, The textured outer surface 19 may be constructed of velour or Dacron™. This textured surface may permit a patient's body to ingrow into regions of the lead assembly covered with this textured surface 19. It may also be noted that the textured surface preferably only coats regions of the lead assembly which necessarily must be anchored to the patient's body. Portions of the relatively thin region 6 which extends externally from the patient's body may not require a textured surface for this reason.

The outer protective layer 21, in this embodiment, performs a similar function of the inner protective sheath 12 described in relation to Figure 2. The outer protective layer 21 adds further wear resistance, maybe flexible, maybe substantially biocompatible and maybe suitable for implantation. The outer protective layer 21 may be constructed of silicone or Nusil™.

Beneath the outer protective layer 21 preferably is a wire bundle 22. This wire bundle 22 may include: three wires 25 (which are insulated preferably by PFA 23), a wiring strain relief 24, and some silicone or Nusil™ to provide dimensional support. The wire bundle 22 may be constructed in similar manner to the wire bundle 14 depicted in Figure 2.

The smaller or thinner diameter of second portion 4 may also increase the anchoring effect of the textured surface, as the thinner region may allow for better tissue integration. The smaller or thinner diameter may be accomplished by the removal of outer protective sheath 1 1 and the shielding layer 13. The shielding layer 13 may not be required for communicating electrical signals with a medical device, particularly in cases where the length of the relatively thin region of the lead assembly is relatively short when compared against the exposed regions of the lead assembly 10 which are external to the patient, such as the first portion 8.

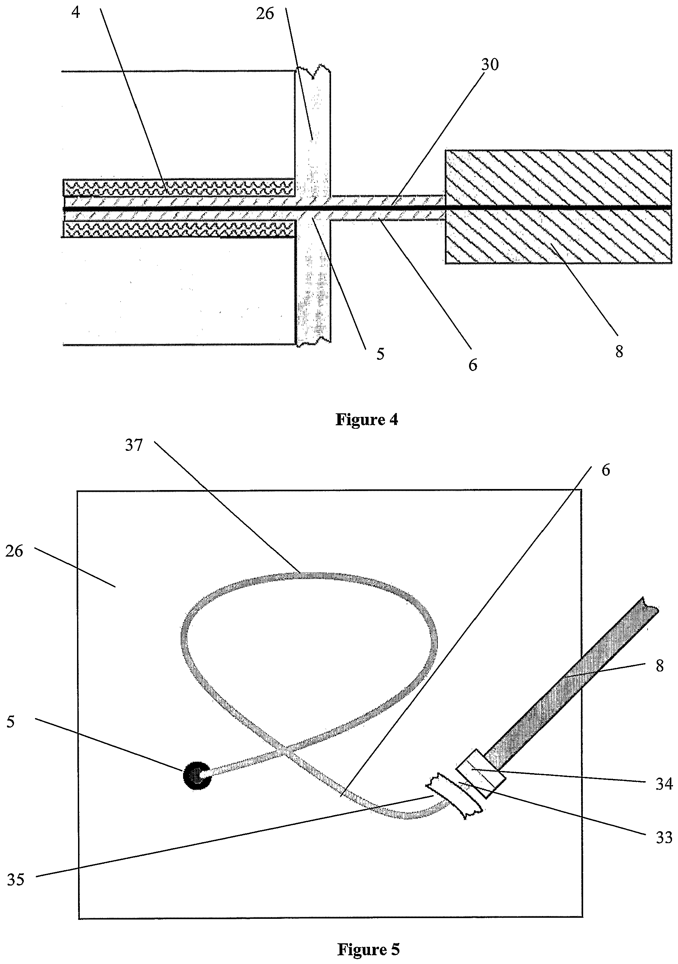

A further embodiment is shown in Figure 4, wherein the lead assembly 10 is implanted within a patient. Please note that similar numerical labelling to Figure 1 has been used in relation to Figure 4. The skin layer 26 of a patient is shown with a hole, aperture or exit wound 5. Preferably, the lead assembly 10 passes through the hole 5. This embodiment depicts the lead assembly 10 including a relatively thin region 6 and the thicker first portion 8 external of the body of the patient. Wires 30 pass through the centre of the lead assembly and allow electrical communication to be achieved between an external device and an internally implanted medical device.

Preferably, the internal portion of the lead assembly includes the relatively thin region 6 coated with a textured surface 4.

Additionally, the size of the hole 5 is minimised because of the thickness of the relatively thin region 6. This minimisation reduces the probability of infection and promotes wound healing by the patient's body.

A further embodiment is shown in Figure 5. In this embodiment, the lead assembly 10 includes a strain terminator mechanism. Figure 5, which uses similar numerical referencing as Figure 1 & 4, shows the external surface of the patient's skin 26 at a site where the lead assembly 10 exits the body. The lead assembly, in this embodiment includes a relatively thin region 6 and a first portion 8, joined by two connectors 33 & 34. These connectors mate to form a connection and allow electrical communication of the wires within the lead assembly.

Preferably, connectors 33 and 34 are submersible and/or water resistance. This water resistance feature will allow the patient to bath, shower or swim in relative safety in regard to medical device failure or electrocution. This may be achieved by including two 'O' rings within the connectors so as to provide a relatively good seal against water penetration. The connectors preferably are made of wear resistance plastic material which is lightweight and unlikely to cause discomfort to the patient. It may also be preferable to allow the connectors to be secured together, when in use, by a screw & thread means.

It may also be preferable for the connectors 33 & 34 to allow for easy replacement of the first portion 8, in situations of accidental breakage without requiring the patient to undergo substantive invasive surgery. This may be achieved by disconnecting the connectors 33 & 34 and then attaching a replacement first portion 8 of the lead assembly.

The strain terminator mechanism includes: a loop of redundant lead 37 and a lead restraint 35. In this embodiment, the loop 37 is formed from the relatively thin region 6 of the lead assembly 10 extending from the hole 5 in the patient's skin layer. The strain relief mechanism is arranged so that if the lead assembly is accidentally or otherwise pulled, the lead assembly is not pulled from the patient's body. Obviously, if the lead assembly was pulled or jerked suddenly the net result may be to cause serious damage to the patient's skin layer and or internal organs. Additionally, the implanted medical device, which the lead assembly is connected internally to, may also be damaged by such an accident or incident.

Preferably, in situations where the lead assembly is pulled the lead restraint 35 would function to dampen the stresses otherwise experienced by hole 5. The lead restraint 35 preferably holds the lead assembly and may at the user's discretion release the lead assembly. The loop 37 of lead assembly functions to supply additional lead if the lead is pulled through the lead restraint 35. The loop 37 serves a backup and provides slack to the lead assembly between the hole 5 and lead restraint 35.

Please note that the loop 37 is not required to be in a loop formation, any redundant lead length (such as a coil of lead) between the lead restraint 35 and the hole 5 will serve a similar function. However the loop formation of the redundant length of lead is generally preferable for presentation or aesthetic reasons.

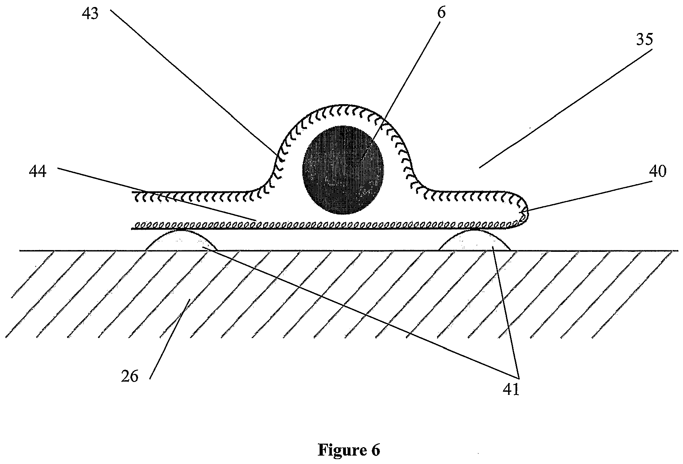

The embodiment shown in Figure 6 depicts a preferred lead restraint 35. This preferred lead restraint 35 includes: a flexible strip 40, interlocking Velcro™ segments 43 & 44 and adhesive 41.

Preferably, the lead restraint 35 is constructed by gluing a portion of the flexible strip 40 to the surface skin layer 26 of a patient. This may be accomplished by applying adhesive 41 to the locations depicted in Figure 6. Attached to the opposed surface of flexible strip 40, which was glued to the patient's skin, may be attached at least two segments of interlocking and complementary Velcro™ 43 & 44 regions. This arrangement preferably allows the flexible strip 40 to fold and allow the complementary Vecro™ 43 & 44 regions to interlock and/or connect.

Preferably, the relatively thin region 6 of the lead assembly 10 is positioned between the two interlocking layers of Velcro™ 43 & 44. The relatively thin region 6 may be secured in place by the lead restraint 35. Preferably, the interlocking regions 43 & 44 secure the relatively thin region 6 firmly enough so as to restrain the lead from accidental stress induced by pulling or stretching. Please note that the lead restraint 35 may be positioned to also restrain the first portion 8.

Various modifications and alterations are possible within the foregoing specification without departing from the scope of this invention.

In an embodiment there is provided a percutaneous lead assembly for supplying electrical signals to a medical device implanted within a body of a patient, said lead assembly comprising a flexible elongate member having a first portion adapted to remain external to the body of a patient, said first portion having a first diameter; and a second portion joined to said first portion and adapted to extend through a hole in a skin layer of the body of the patient, and wherein said second portion having a second diameter which is substantially smaller than said first diameter.

The first portion includes a shielding layer and/or at least a segment of said second portion is covered with a textured surface. The first portion and said second portion are joined by connectors. The percutaneous lead assembly may include a lead restraint.

In an embodiment there is provided an external lead restraint for use with a percutaneous lead, wherein said lead is implanted within a body of a patient and extends through a hole in the patient's skin and characterised in that an excess length of lead is releasably secured near to the hole by releasable securing means affixed to the patient's skin.

In an embodiment there is provided a percutaneous lead assembly for supplying electrical signal to a medical device implanted within a body of a patient, wherein said lead assembly has a flexible elongate member including a first unshielded portion that extends through a hole in a skin layer of the body of the patient; and a second shielded portion which is joined to said first unshielded portion at a site external to the body of the patient.

## Claims

1. A percutaneous lead assembly for supplying electrical signals to a medical device implanted within a body of a patient, said lead assembly comprising
a flexible elongate member having
a first portion (8) adapted to remain external to the body of a patient and
a second portion (4) joined to said first portion (8);
wherein the first portion (8) and the second portion (4) include respective first (34) and second (33) connectors adapted to mate and allow electrical communication within the percutaneous lead assembly (10),
wherein the first portion (8) has a first diameter and the second portion (4) has a second diameter which is substantially smaller than the first diameter;
wherein a segment of the second portion (4) is covered with a textured surface (19), and
wherein the second portion (4) includes an unshielded portion that extends through a hole (5) in a skin layer (26) of the body of the patient; and the first portion (8) includes a shielded portion which is joined to the unshielded portion at a site external to the body of the patient when the connectors (33, 34) are mated.

2. The percutaneous lead assembly as claimed in claim 1, wherein the connectors further allow for removal of the first portion (8) from the second portion (4).

3. The percutaneous lead assembly as claimed in claim 1, wherein the percutaneous lead assembly (10) includes a lead restraint (35).

4. The percutaneous lead assembly as claimed in claim 3, wherein when the second portion (4) is implanted within the body of the patient and the unshielded portion extends through the hole (5) in the patient's skin (26), an excess length (37) of lead is releasably secured near to the hole (5) by releasable securing means affixed to the patient's skin.

5. The percutaneous lead assembly as claimed in claim 2, wherein the first (34) and second (33) connectors are mated by a screw and thread means.

6. The percutaneous lead assembly as claimed in claim5, wherein O rings are included within the first (33) and second (34) connectors to make them submersible or water resistant.

7. The percutaneous lead assembly as claimed in claim 1, wherein a wire bundle (14, 22) comprising three insulated wires (15, 25) and a wiring strain relief (17, 24) are contained within the percutaneous lead assembly (10).

8. The percutaneous lead assembly as claimed in any of the previous claims, wherein the textured surface (19) is a coating of velour or dacron.

## Patentansprüche

1. Eine perkutane Leitungsanordnung zur Versorgung von einer in einem Körper eines Patienten implantierten medizinischen Vorrichtung mit elektrischen Signalen, wobei die Leitungsanordnung folgendes umfasst
ein flexibles langgestrecktes Element, welches folgendes hat
einen ersten Teil (8), der angepasst ist, um außerhalb des Körpers eines Patienten angeordnet zu bleiben und
einen zweiten Teil (4), der mit dem ersten Teil (8) verbunden ist;
wobei der erste Teil (8) und der zweite Teil (4) entsprechende erste (34) und zweite (33) Verbindungsstücke umfassen, die angepasst sind, um ineinander zu greifen und um eine elektrische Verbindung mit der perkutanen Leitungsanordnung (10) zu ermöglichen,
wobei der erste Teil (8) einen ersten Durchmesser hat und der zweite Teil (4) einen zweiten Durchmesser hat, der im Wesentlichen kleiner als der erste Durchmesser ist;
wobei ein Abschnitt des zweiten Teils (4) mit einer strukturierten Oberfläche (19) gedeckt ist, und
wobei der zweite Teil (4) einen ungeschirmten Teil umfasst, der sich durch eine Bohrung (5) in einer Hautschicht (26) des Körpers des Patienten erstreckt; und wobei der erste Teil (8) einen geschirmten Teil umfasst, der mit dem ungeschirmten Teil an einer Stelle außerhalb des Körpers des Patienten verbunden ist, wenn die Verbindungsstücke (33, 34) ineinander greifen.

2. Die perkutane Leitungsanordnung wie in Anspruch 1 beansprucht, wobei die Verbindungsstücke weiterhin die Entfernung des ersten Teils (8) vom zweiten Teil (4) ermöglichen.

3. Die perkutane Leitungsanordnung wie in Anspruch 1 beansprucht, wobei die perkutane Leitungsanordnung (10) eine Leitungsarretierung (35) umfasst.

4. Die perkutane Leitungsanordnung wie in Anspruch 3 beansprucht, wobei, wenn der zweite Teil (4) im Körper des Patienten implantiert ist und sich der ungeschirmte Teil durch die Bohrung (5) in die Patientenhaut (26) hinein erstreckt, eine Überlänge (37) der Leitung in der Nähe der Bohrung (5) mittels eines an der Patientenhaut angebrachten lösbaren Befestigungsmittels lösbar befestigt ist.

5. Die perkutane Leitungsanordnung wie in Anspruch 2 beansprucht, wobei das erste (34) und das zweite (33) Verbindungsstück mit Hilfe von einem aus Schraube und Gewinde bestehenden Mittel ineinandergreifen.

6. Die perkutane Leitungsanordnung wie in Anspruch 5 beansprucht, wobei O-Ringe innerhalb des ersten (33) und des zweiten (34) Verbindungsstücks enthalten sind, damit diese tauchfähig oder wasserfest sind.

7. Die perkutane Leitungsanordnung wie in Anspruch 1 beansprucht, wobei ein Drahtbündel (14, 22) umfassend drei isolierte Drähte (15, 25) und eine Drahtzugentlastung (17, 24) innerhalb der perkutanen Leitungsanordnung (10) enthalten sind.

8. Die perkutane Leitungsanordnung wie in einem der vorhergehenden Ansprüche beansprucht, wobei die strukturierte Oberfläche (19) eine Velours- oder Dacronbeschichtung ist.

## Revendications

1. Un ensemble conducteur percutané pour fournir des signaux électriques à un dispositif médical implanté dans un corps d'un patient, ledit ensemble conducteur comprenant
un élément allongé flexible ayant
une première partie (8) adaptée pour rester hors du corps d'un patient et
une seconde partie (4) reliée à ladite première partie (8) ;
dans lequel la première partie (8) et la seconde partie (4) incluent des connecteurs respectifs premier (34) et second (33) adaptés pour s'accoupler et permettre une communication électrique dans l'ensemble conducteur percutané (10),
dans lequel la première partie (8) a un premier diamètre et la seconde partie (4) a un second diamètre qui est essentiellement plus petit que le premier diamètre ;
dans lequel un segment de la seconde partie (4) est recouvert avec une surface texturée (19), et
dans lequel la seconde partie (4) inclut une partie non protégée qui s'étend à travers une ouverture (5) dans une couche de la peau (26) du corps du patient ; et la première partie (8) inclut une partie protégée qui est reliée à la partie non protégée dans un endroit extérieure au corps du patient lorsque les connecteurs (33, 34) sont accouplés.

2. L'ensemble conducteur percutané tel que revendiqué dans la revendication 1, dans lequel les connecteurs permettent en outre d'enlever la première partie (8) de la seconde partie (4).

3. L'ensemble conducteur percutané tel que revendiqué dans la revendication 1, dans lequel l'ensemble conducteur percutané (10) inclut un dispositif de retenue du conducteur (35).

4. L'ensemble conducteur percutané tel que revendiqué dans la revendication 3, dans lequel, lorsque la seconde partie (4) est implantée dans le corps du patient et la partie non protégée s'étend à travers l'ouverture (5) dans la peau du patient (26), une longueur en excès (37) du conducteur est attachée de façon amovible près de l'ouverture (5) moyennant un moyen d'attache amovible fixé à la peau du patient.

5. L'ensemble conducteur percutané tel que revendiqué dans la revendication 2, dans lequel les connecteurs premier (34) et second (33) sont accouplés avec un moyen de vissage et enfilage.

6. L'ensemble conducteur percutané tel que revendiqué dans la revendication 5, dans lequel des juntes toriques sont incluses dans les connecteurs premier (33) et second (34) afin de les faire submersibles ou résistants à l'eau.

7. L'ensemble conducteur percutané tel que revendiqué dans la revendication 1, dans lequel un faisceau de fils (14, 22) comprenant trois fils isolés (15, 25) et un moyen de soulagement de tension de fils (17, 24) sont contenus dans l'ensemble conducteur percutané (10).

8. L'ensemble conducteur percutané tel que revendiqué dans l'une quelconque des revendications précédentes, dans lequel la surface texturée (19) est un revêtement de velours ou de dacron.
